# EUROPEAN PATENT APPLICATION

(11) **EP 4 404 135 A1**
(43) Date of publication of application: **24.07.2024**
(21) Application number: 23712459.9
(22) Date of filing: 17.03.2023
(51) Int. Cl.: G06T 7/00

(54) **ACCESSORY APPLICATION AND CONTROL SYSTEM FOR SIMPLIFYING ORAL IMPLANT RESTORATION PROCESS**

(30) Priority: 05.12.2022 CN 202211545854
(71) Applicant: Gaofeng Medical Equipment (Wuxi) Co., Ltd, Huishan Economic Development Zone Wuxi, Jiangsu 214000 (CN)
(72) Inventor: GAO, Xingrong, Wuxi, Jiangsu 214000 (CN); BAO, Yaxing, Wuxi, Jiangsu 214000 (CN)
(74) Representative: Jannig & Repkow Patentanwälte PartG mbB
(86) International application number: PCT/CN2023/082122
(87) International publication number: WO 2024/119661

(57) **Abstract**

The present invention discloses an accessory application and regulation system for simplifying an dental implant restoration process, which comprises a dental collection module, a dental analysis module, a dental data collection module, a dental data processing module, an accessory matching module, an accessory information collection module, an accessory information analysis module, an accessory installation collection module and an accessory installation evaluation module, wherein the dental collection module is used for collecting dental image information before accessory installation and continuously collecting the dental image information for three times, the dental analysis module is used for analyzing the dental image information to obtain dental evaluation information, and the dental analysis module is used for analyzing the dental image information to obtain the dental evaluation information. The present invention is better able to take the fitting degree of the accessories into account, which is for enhancing the installation and assembly experience, namely, better protecting the person to be restored, and for better accessory application and regulation.

## Description

### Technical Field

The present invention relates to the field of dental restoration, in particular to an accessory application and regulation system for simplifying a dental implant restoration process.

### Background Art

The whole process of dental implant restoration, is that after the implant is implanted, the entire restoration is fitted into the patients oral cavity. In the process, the patient wears an accessory after the implant is implanted for gingival healing and engagement. The oral cavity is directly scanned, or the impression is directly taken, the data are imported into the dental design software, and the corresponding data are selected to design the restoration. After the design is completed, it is processed and manufactured according to the product requirements, the teeth are fitted after completion, and the base cover is taken out, and then the restoration is fitted; and
during the installation of protective accessories, it is necessary to use the accessory application and regulation system to regulate and monitor the application of accessories.

In actual use of the existing accessory application and regulation system, the suitability of the accessories is not considered, and the effect of applying and deploying the accessories is poor, which has a certain impact on the use of the accessory application and regulation system. Therefore, an accessory application and regulation system for simplifying dental implant restoration process is proposed.

### Summary of the Invention

The technical problem to be solved by the present invention is that how to solve the problem that the existing accessories application and regulation system does not consider the suitability of the accessories in the process, and the poor effect of application and deployment of accessories brings a certain impact on the use of the accessory application and regulation system, and an accessory application and regulation system for simplifying dental implant restoration process is provided.

The present invention solves the above-mentioned technical problems through the following technical solutions, and the present invention comprising:
a dental collection module, the dental collection module is applicable to collect dental image information prior to installing accessories, by continuously collecting dental image information thrice;
a dental analysis module, the dental analysis module is applicable to analyze the dental image information to obtain dental evaluation information;
a dental data collection module, the dental data collection module is applicable to scan tooth data in an oral cavity by means of an optical scanning device;
a dental data processing module, the dental data processing module is applicable to process dental data scanned by the optical scanning device in the dental to obtain preliminary model information;
an accessory matching module, the accessory matching module is applicable to process the collected preliminary model information to generate matching parameter information, and match the matching parameter information with all accessories in an accessory library to obtain matching accessory information;
after receiving the matching accessory information, the accessory application and regulation system retrieves a preset number of the corresponding matching accessories from the accessory library;
an accessory information collection module, the accessory information collection module is applicable to collect a preset number of the corresponding matching accessory image information, and to perform enhanced processing on the matching accessory image information to obtain high-definition accessory image information;
an accessory information analysis module, the accessory information analysis module is applicable to analyze and process the high-definition accessory image information to obtain accessory evaluation information;
an accessory installation collection module, the accessory installation collection module is applicable to collect the image information after the accessories are installed, and to process the image information after the accessories are installed to obtain the high-definition installation information; and
an accessory installation evaluation module, the accessory installation module is applicable to process the high-definition installation information to generate accessory installation adjustment information and accessory installation completion information.

Further, a process of collecting dental image information before installing the accessories by the dental collection module comprises: prior to dental image collection, a camera of an image collection device is heated, and after temperature of the camera is increased to a preset value, a camera of the image collection device is extended into the oral cavity and is moved in a raster pattern at a constant speed, and continuously collects the dental image information thrice.

Further, the dental evaluation information comprises suitable installation information and unsuitable installation information, and the dental analysis module analyzes the dental image information to obtain the specific processing process of the dental evaluation information comprises: the three collected dental image information is extracted, definition of the three dental image information is first analyzed, the dental image information with the highest definition as an analysis image is extracted, color of the analysis image is extracted, when area of the preset color in the analysis image is greater than a preset value, the suitable installation information is generated, and when the area of the preset color in the analysis image is less than the preset value, the unsuitable installation information is generated.

Further, a specific process of matching the matching parameter information with all accessories in the accessory library to obtain the matching accessory information comprises: the obtained matching parameter information is extracted, the matching parameter information is marked as K, the accessory parameter information of all the accessories in the accessory library is marked as Qi, i being the accessory parameter information, and a difference value between the matching parameter information K and the accessory parameter information Qi of all the accessories is sequentially calculated, thus i number of Kq_{difference} is obtained; an accessory corresponding to a minimum absolute value of the i Kq_{difference} is extracted as the matching accessory information.

Further, a processing process of obtaining the high-definition accessory image information by perform enhanced processing on the matching accessory image information comprises: the image is enhanced by a spatial domain method to obtain a first enhanced image, the image is enhanced by a frequency domain method to obtain a second enhanced image, and the image is enhanced by a color image enhancement method to obtain a third enhanced image, and the first enhanced image, the second enhanced image and the third enhanced image are ranked from high to low according to the definition, and an image with the highest definition is extracted as high-definition accessory image information.

Further, the spatial domain method comprises a point operation method and a template processing method, the point operation process comprises gray scale transformation, a histogram modification method and a local statistical method, and a process of the template processing method comprises image smoothing and image sharpening;
the frequency domain method comprises high-pass filtering, low-pass filtering and homomorphic filtering; and
the color image enhancement method comprises pseudo-color enhancement, false color enhancement, and true color enhancement.

Further, the accessory evaluation information comprises normal accessory information and abnormal accessory information, and a specific process of analyzing and processing the high-definition accessory image information obtained by the accessory information analysis module comprises: the obtained high-definition accessory image information is analyzed and processed, and the number of abnormal protrusions on the accessories and deviation between the number of cracks and the standard size are obtained, when either the number of the abnormal protrusions on the accessories is greater than a preset value or the number of the cracks is greater than the deviation between the preset value and the standard size occurs, the abnormal accessory information is generated, and when neither the number of the abnormal protrusions is greater than the preset value nor the number of the cracks is greater than the deviation between the preset value and the standard size occurs, the normal accessory information is generated.

Further, a specific process of processing the high-definition installation information to generate accessory installation adjustment information and accessory installation completion information by the accessory installation module comprises: the collected high-definition installation information is extracted, real-time installation height and installation angle are obtained from the high-definition installation information, the estimated installation height and estimated installation angle are further extracted, when a difference value between the real-time installation height and the estimated installation height is less than a preset value, and a difference value between the real-time installation angle and the estimated installation angle is less than a preset value, installation completion information of the accessories is generated, and when the difference value between the real-time installation height and the estimated installation height is greater than the preset value, or the difference value between the real-time installation angle and the estimated installation angle is greater than the preset value, installation adjustment information of the accessories is generated.

Compared with the prior art, the present invention has the following advantages: the accessory application and regulation system for simplifying dental implant restoration process collects the state of the oral cavity before installing the accessories, and determines whether it is in a state suitable for installation, so as to avoid the occurrence of displacement of accessories after installation caused by poor dental condition, and at the same time, through the overall evaluation of the status of the accessories before installation, whether there are defects in the accessories can be known, which effectively prevents accidents caused by the installation of defective accessories, and ensures safety of the accessories. At the same time, the overall evaluation of the installation status of the accessories is carried out after ensuring that they are safe, and when it is found that the installation of the accessories is not in place, timely reminders are generated to adjust the position of the accessories to ensure the accuracy of the installation of the accessories. Through the above process, the system can have more functions, which meets the actual needs of different uses, and can more accurately apply and deploy accessories simplifying the dental implant restoration process, and can make the system more worthy of promotion and use.

### Description of Drawings

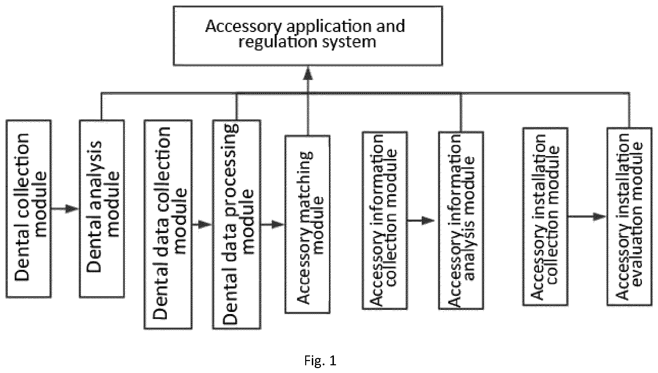

Fig. 1 is a system block diagram of the present invention.

### Specific Embodiments

Hereinafter, embodiments of the present invention are described in detail, and the embodiments of the present invention are implemented on the premise of the technical solutions of the present invention, and detailed embodiments and specific operation processes are provided, but the protection scope of the present invention is not limited to the following embodiments.

As shown in Fig. 1, the present embodiment provides a technical solution: an accessory application and regulation system for simplifying dental implant restoration process, comprising:
a dental collection module, the dental collection module is applicable to collect dental image information prior to installing accessories , by continuously collecting dental image information thrice;
a dental analysis module, the dental analysis module is applicable to analyze the dental image information to obtain dental evaluation information;
a dental data collection module, the dental data collection module is applicable to scan tooth data in an oral cavity by means of an optical scanning device;
a dental data processing module, the dental data processing module is applicable to process dental data scanned by the optical scanning device in the dental to obtain preliminary model information;
an accessory matching module, wherein the accessory matching module is applicable to process the collected preliminary model information to generate matching parameter information, and match the matching parameter information with all accessories in an accessory library to obtain matching accessory information;
after receiving the matching accessory information, the accessory application and regulation system retrieves a preset number of the corresponding matching accessories from the accessory library;
an accessory information collection module, the accessory information collection module is applicable to collect a preset number of the corresponding matching accessory image information, and to perform enhanced processing on the matching accessory image information to obtain high-definition accessory image information;
an accessory information analysis module, the accessory information analysis module is applicable to analyze and process the high-definition accessory image information to obtain accessory evaluation information;
an accessory installation collection module, the accessory installation collection module is applicable to collect the image information after the accessories are installed, and to process the image information after the accessories are installed to obtain the high-definition installation information; and
an accessory installation evaluation module, the accessory installation module is applicable to process the high-definition installation information to generate accessory installation adjustment information and accessory installation completion information.

A process of collecting dental image information before installing the accessories by the dental collection module comprises: prior to dental image collection, a camera of an image collection device is heated, and after temperature of the camera is increased to a preset value, a camera of the image collection device is extended into the oral cavity and is moved in a raster pattern at a constant speed, and continuously collects the dental image information thrice.

Through the above process, clarity of the collected images in the dental can be guaranteed, and the analysis of the dental image information with higher definition can analyze the state information in the dental more accurately, so that the situation of large deviation of the analysis information caused by the low definition of the dental images can be effectively avoided.

The dental evaluation information comprises suitable installation information and unsuitable installation information, and the dental analysis module analyzes the dental image information to obtain the specific processing process of the dental evaluation information comprises: the three collected dental image information is extracted, definition of the three dental image information is first analyzed, the dental image information with the highest definition as an analysis image is extracted, color of the analysis image is extracted, when area of the preset color in the analysis image is greater than a preset value, the suitable installation information is generated, and when the area of the preset color in the analysis image is less than the preset value, the unsuitable installation information is generated.

The preset colors comprise red. If the red area is too large, it means that the bleeding in the oral cavity is serious and it is not suitable for installation. Otherwise, it will generate suitable installation information. Through the above process, it can be determined whether to install accessories according to the dental state of the person to be restored, which can better ensure stability of the installation of accessories, reduce the pain of the person to be restored, prevent the position of the fitting from falling off during the recovery process caused by forced installation of accessories due to poor dental condition, and better protect the person to be restored.

A specific process of matching the matching parameter information with all accessories in the accessory library to obtain the matching accessory information comprises: the obtained matching parameter information is extracted, the matching parameter information is marked as K, the accessory parameter information of all the accessories in the accessory library is marked as Qi, i being the accessory parameter information, and a difference value between the matching parameter information K and the accessory parameter information Qi of all the accessories is sequentially calculated, thus i number of Kq_{difference} is obtained; an accessory corresponding to a minimum absolute value of the i Kq_{difference} is extracted as the matching accessory information.

Through the above process, it is possible to obtain installation accessories that are more suitable for the person to be installed, which improves the installation experience, effectively reduces the occurrence of strong foreign body sensation in the dental of the person to be restored after installation caused by unsuitable size of the accessories, and also effectively prevent the occurrence of the situation of large restoration deviation caused by the unsuitable size, ensuring the accuracy of restoration.

A processing process of obtaining the high-definition accessory image information by perform enhanced processing on the matching accessory image information comprises: the image is enhanced by a spatial domain method to obtain a first enhanced image, the image is enhanced by a frequency domain method to obtain a second enhanced image, and the image is enhanced by a color image enhancement method to obtain a third enhanced image, and the first enhanced image, the second enhanced image and the third enhanced image are ranked from high to low according to the definition, and an image with the highest definition is extracted as high-definition accessory image information.

Through the above process, clearer image information can be obtained, so that more accurate evaluation and analysis can be carried out. For clearer image information, fine defects on matching accessories can be found, thereby reducing the occurrence of the situation where the fine defects on the matching accessories are not found.

The spatial domain method comprises a point operation method and a template processing method, the point operation process comprises gray scale transformation, a histogram modification method and a local statistical method, and a process of the template processing method comprises image smoothing and image sharpening;
the frequency domain method comprises high-pass filtering, low-pass filtering and homomorphic filtering; and
the color image enhancement method comprises pseudo-color enhancement, false color enhancement, and true color enhancement.

Through the above method, clarity of the obtained images can be effectively improved, thereby ensuring the accuracy of subsequent result analysis.

The accessory evaluation information comprises normal accessory information and abnormal accessory information, and a process of analyzing and processing the high-definition accessory image information obtained by the accessory information analysis module is comprises: the obtained high-definition accessory image information is analyzed and processed, and the number of abnormal protrusions on the accessories and deviation between the number of cracks and the standard size are obtained, when either the number of the abnormal protrusions on the accessories is greater than a preset value or the number of the cracks is greater than the deviation between the preset value and the standard size occurs, the abnormal accessory information is generated, and when neither the number of the abnormal protrusions is greater than the preset value nor the number of the cracks is greater than the deviation between the preset value and the standard size occurs, the normal accessory information is generated.

Through the above process, it is possible to find out whether there are defects in the installed accessories in time, which effectively avoids accidents caused by the installation of defective accessories, prevents accidents such as defective products being damaged in the dental after the defective products are installed, and better protect safety of the dental of the person to be restored.

A process of processing the high-definition installation information to generate accessory installation adjustment information and accessory installation completion information by the accessory installation module comprises: the collected high-definition installation information is extracted, real-time installation height and installation angle are obtained from the high-definition installation information, the estimated installation height and estimated installation angle are further extracted, when a difference value between the real-time installation height and the estimated installation height is less than a preset value, and a difference value between the real-time installation angle and the estimated installation angle is less than a preset value, installation completion information of the accessories is generated, and when the difference value between the real-time installation height and the estimated installation height is greater than the preset value, or the difference value between the real-time installation angle and the estimated installation angle is greater than the preset value, installation adjustment information of the accessories is generated.

Through the above process, when it is found that the installation of the accessories is not in place, timely reminders are generated to adjust the position of the accessories to ensure the accuracy of the installation of the accessories, which effectively avoids the occurrence of the situation of re-restoration caused by the undiscovered deviation in the installation of accessories, and further reduces the restoration burden of the person to be restored.

When the present invention is in use, the state of the oral cavity before installing the accessories is collected, and whether it is in a state suitable for installation is determined, so as to avoid the occurrence of displacement of accessories after installation caused by poor dental condition, and at the same time, through the overall evaluation of the status of the accessories before installation, whether there are defects in the accessories can be known, which effectively prevents accidents caused by the installation of defective accessories, and ensures safety of the accessories. At the same time, the overall evaluation of the installation status of the accessories is carried out after ensuring that they are safe, and when it is found that the installation of the accessories is not in place, timely reminders are generated to adjust the position of the accessories to ensure the accuracy of the installation of the accessories. Through the above process, the system can have more functions, which meets the actual needs of different uses, and can more accurately apply and deploy accessories simplifying the dental implant restoration process, and can make the system more worthy of promotion and use.

In addition, the terms "first" and "second" are used for descriptive purposes only, and cannot be interpreted as indicating or implying relative importance or implicitly specifying the quantity of indicated technical features. Thus, the features defined as "first" and "second" may explicitly or implicitly comprise at least one of these features. In the description of the present invention, "plurality" means at least two, such as two, three, etc., unless otherwise specifically defined.

In the description of the present specification, descriptions referring to the terms "one embodiment", "some embodiments", "example", "specific examples", or "some examples" mean that specific features described in connection with the embodiment or example, structure, material or characteristic is included in at least one embodiment or example of the present invention. In the present specification, the schematic representations of the above terms are not necessarily directed to the same embodiment or example. Furthermore, the described specific features, structures, materials or characteristics may be combined in any suitable manner in any one or more embodiments or examples. In addition, those skilled in the art can combine and compose different embodiments or examples and features of different embodiments or examples described in the present specification without conflicting with each other.

Although the embodiments of the present invention have been shown and described above, it can be understood that the above embodiments are exemplary and should not be regarded as limitation to the present invention, and those skilled in the art can make changes, modifications, substitutions and modifications to the above-mentioned embodiments within the scope of the present invention.

## Claims

1. An accessory application and regulation system for simplifying dental implant restoration process, comprising:
a dental collection module, the dental collection module is applicable to collect dental image information prior to installing accessories, by continuously collecting dental image information thrice;
a dental analysis module, the dental analysis module is applicable to analyze the dental image information to obtain dental evaluation information;
a dental data collection module, the dental data collection module is applicable to scan tooth data in an oral cavity by means of an optical scanning device;
a dental data processing module, the dental data processing module is applicable to process dental data scanned by the optical scanning device in the dental to obtain preliminary model information;
an accessory matching module, wherein the accessory matching module is applicable to process the collected preliminary model information to generate matching parameter information, and match the matching parameter information with all accessories in an accessory library to obtain matching accessory information;
after receiving the matching accessory information, the accessory application and regulation system retrieves a preset number of the corresponding matching accessories from the accessory library;
an accessory information collection module, the accessory information collection module is applicable to collect a preset number of the corresponding matching accessory image information, and to perform enhanced processing on the matching accessory image information to obtain high-definition accessory image information;
an accessory information analysis module, the accessory information analysis module is applicable to analyze and process the high-definition accessory image information to obtain accessory evaluation information;
an accessory installation collection module, the accessory installation collection module is applicable to collect the image information after the accessories are installed, and to process the image information after the accessories are installed to obtain the high-definition installation information; and
an accessory installation evaluation module, the accessory installation module is applicable to process the high-definition installation information to generate accessory installation adjustment information and accessory installation completion information.

2. The accessory application and regulation system for simplifying dental implant restoration process according to claim 1, wherein a process of collecting dental image information before installing the accessories by the dental collection module comprises: prior to dental image collection, a camera of an image collection device is heated, and after temperature of the camera is increased to a preset value, a camera of the image collection device is extended into the oral cavity and is moved from left to right and from top to bottom at a constant speed, and continuously collects the dental image information thrice.

3. The accessory application and regulation system for simplifying dental implant restoration process according to claim 1, wherein the dental evaluation information comprises suitable installation information and unsuitable installation information, and the dental analysis module analyzes the dental image information to obtain the specific processing process of the dental evaluation information comprises: the three collected dental image information is extracted, definition of the three dental image information is first analyzed, the dental image information with the highest definition as an analysis image is extracted, color of the analysis image is extracted, when area of the preset color in the analysis image is greater than a preset value, the suitable installation information is generated, and when the area of the preset color in the analysis image is less than the preset value, the unsuitable installation information is generated.

4. The accessory application and regulation system for simplifying dental implant restoration process according to claim 1, wherein a process of matching the matching parameter information with all accessories in the accessory library to obtain the matching accessory information comprises: the obtained matching parameter information is extracted, the matching parameter information is marked as K, the accessory parameter information of all the accessories in the accessory library is marked as Qi, i being the accessory parameter information, and a difference value between the matching parameter information K and the accessory parameter information Qi of all the accessories is sequentially calculated, thus i number of Kq_{difference} is obtained; an accessory corresponding to a minimum absolute value of the i Kq_{difference} is extracted as the matching accessory information.

5. The accessory application and regulation system for simplifying dental implant restoration process according to claim 1, wherein a processing process of obtaining the high-definition accessory image information by perform enhanced processing on the matching accessory image information comprises: the image is enhanced by a spatial domain method to obtain a first enhanced image, the image is enhanced by a frequency domain method to obtain a second enhanced image, and the image is enhanced by a color image enhancement method to obtain a third enhanced image, and the first enhanced image, the second enhanced image and the third enhanced image are ranked from high to low according to the definition, and an image with the highest definition is extracted as high-definition accessory image information.

6. The accessory application and regulation system for simplifying dental implant restoration process according to claim 5, wherein the spatial domain method comprises a point operation method and a template processing method, the point operation process comprises gray scale transformation, a histogram modification method and a local statistical method, and a process of the template processing method comprises image smoothing and image sharpening;
the frequency domain method comprises high-pass filtering, low-pass filtering and homomorphic filtering; and
the color image enhancement method comprises pseudo-color enhancement, false color enhancement, and true color enhancement.

7. The accessory application and regulation system for simplifying dental implant restoration process according to claim 1, wherein the accessory evaluation information comprises normal accessory information and abnormal accessory information, and a process of analyzing and processing the high-definition accessory image information obtained by the accessory information analysis module comprises: the obtained high-definition accessory image information is analyzed and processed, and the number of abnormal protrusions on the accessories and deviation between the number of cracks and the standard size are obtained, when either the number of the abnormal protrusions on the accessories is greater than a preset value or the number of the cracks is greater than the deviation between the preset value and the standard size occurs, the abnormal accessory information is generated, and when neither the number of the abnormal protrusions is greater than the preset value nor the number of the cracks is greater than the deviation between the preset value and the standard size occurs, the normal accessory information is generated.

8. The accessory application and regulation system for simplifying dental implant restoration process according to claim 1, wherein a process of processing the high-definition installation information to generate accessory installation adjustment information and accessory installation completion information by the accessory installation module comprises: the collected high-definition installation information is extracted, real-time installation height and installation angle are obtained from the high-definition installation information, the estimated installation height and estimated installation angle are further extracted, when a difference value between the real-time installation height and the estimated installation height is less than a preset value, and a difference value between the real-time installation angle and the estimated installation angle is less than a preset value, installation completion information of the accessories is generated, and when the difference value between the real-time installation height and the estimated installation height is greater than the preset value, or the difference value between the real-time installation angle and the estimated installation angle is greater than the preset value, installation adjustment information of the accessories is generated.
